# EUROPEAN PATENT APPLICATION

(11) **EP 3 096 139 A1**
(43) Date of publication of application: **23.11.2016**
(21) Application number: 15737831.6
(22) Date of filing: 14.01.2015
(51) Int. Cl.: G01N 33/543, G01N 21/78

(54) **IMMUNOCHROMATOGRAPHIC ANALYSIS METHOD, IMMUNOCHROMATOGRAPHIC ANALYSIS DEVICE AND IMMUNOCHROMATOGRAPHIC ANALYSIS KIT**

(30) Priority: 14.01.2014 JP 2014004289
(71) Applicant: Tanaka Kikinzoku Kogyo K.K., Chiyoda-ku Tokyo 100-6422 (JP)
(72) Inventor: ITO, Daisuke, Hiratsuka-shi Kanagawa 254-0076 (JP); NAKAJIMA, Satoru, Hiratsuka-shi Kanagawa 254-0076 (JP); SHIBAI, Yusuke, Hiratsuka-shi Kanagawa 254-0076 (JP)
(74) Representative: Peguet, Wilfried
(86) International application number: PCT/JP2015/050838
(87) International publication number: WO 2015/108082

(57) **Abstract**

Provided is an immunochromatographic analysis method, which does not require expert knowledge of a specific device or analysis and also does not involve complicated operations by individually causing a first substance to be detected, which is to be determined to be positive or negative, and a second substance to be detected, which is used as a standard for making a positive or negative determination by ascertaining the ratio of the second substance to be detected to the first substance to be detected, to develop color by an immune reaction, and setting the concentration at which the strengths of the developed color signals of the two substances to be detected are the same as a boundary for the determination of the first substance to be detected.

## Description

### TECHNICAL FIELD

The present invention relates to an immunochromatographic analysis method, an immunochromatographic analysis device, and an immunochromatographic analysis kit.

### BACKGROUND ART

Measurement of various components contained in a specimen such as blood or urine is clinically extremely important for ascertaining the physical conditions of a patient, and conventionally, various measurement methods are adopted according to the components. As one of the methods, there has been known an immunochromatographic analysis method, in which a substance to be detected contained in a specimen is caused to develop color by an immune reaction, and the developed color signal is confirmed.

On the other hand, there were 366 million diabetic patients in the world in 2011, and the number of diabetic patients in the world is expected to reach 552 million (about 10% of the adult population) in 2030. Further, the number of so-called prediabetic individuals is considered to be equal to or more than the number of diabetic patients.

Conventionally, the diagnosis of diabetes has been made by measuring the blood glucose level. However, recently, the blood level of glycated hemoglobin (glycohemoglobin) in which a sugar is bound to hemoglobin in the blood, particularly hemoglobin A1c (hereinafter referred to as "HbA1c") in which the N-terminal valine residue of the hemoglobin β chain has been glycated reflects the average blood glucose level in the past 1 to 2 months, and therefore has begun to be used as an index suitable for diagnosis of diabetes or follow-up observation of diabetes (see, for example, Patent Document 1).

The measurement of HbA1c is conventionally performed by an HPLC method, a capillary electrophoresis method, an enzymatic method, an immunological measurement method, or the like, however, these methods require expert knowledge of a specific device or analysis, and therefore have a problem that the value of HbA1c cannot be easily known in small-sized hospitals, at home, etc.

Further, with respect to the blood level of HbA1c, there are individual differences in blood components, and therefore, a positive or negative determination of diabetes is made by simultaneously measuring hemoglobin which is not glycated (hereinafter referred to as "HbA0"), and ascertaining the ratio of HbA1c to HbAO, however, in this case, it is necessary to individually measure HbA1c and HbA0 by the above-mentioned method, and therefore, there is also a problem that the complexity of the measurement is further increased.

In addition, the components other than HbA1c, for example, PSA (free PSA/total PSA ratio), cholesterol (LDL cholesterol/HDL cholesterol ratio) serving as the index of arteriosclerosis, a marker for hepatic dysfunction, nephrotic syndrome, or the like (albumin/globulin ratio), and the like are also important factors for ascertaining the physical conditions of a patient. However, also these components have the same problem as the above-mentioned HbA1c. That is, the measurement of the component requires expert knowledge of a specific device or analysis, and also it is necessary to simultaneously measure a reference substance such as HbAO for accurately confirming the component to be positive or negative, and therefore, there is a problem that the measurement is practically impossible in small-sized hospitals, at home, etc.

### CITED REFERENCES

### PATENT DOCUMENT

Patent Document 1: JP-A-2012-251789

### DISCLOSURE OF THE INVENTION

### PROBLEMS TO BE SOLVED BY THE INVENTION

In view of this, an object of the present invention is to solve the above-mentioned conventional problems and provide an immunochromatographic analysis method, an immunochromatographic analysis device, and an immunochromatographic analysis kit, capable of measuring various components contained in various specimens such as blood and urine even in small-sized hospitals, at home, etc. without requiring expert knowledge of a specific device or analysis and also without involving complicated operations.

### MEANS FOR SOLVING THE PROBLEMS

As a result of intensive studies, the present inventors found that the conventional problems as described above can be solved by individually causing a first substance to be detected, which is to be determined to be positive or negative, and a second substance to be detected, which is used as a standard for making a positive or negative determination by ascertaining the ratio of the second substance to be detected to the first substance to be detected, to develop color by an immune reaction, and setting the concentration at which the strengths of the developed color signals of the two substances to be detected are the same as a boundary for the determination of the first substance to be detected, and thus, the present invention could be completed.

That is, the present invention is as follows.
1. An immunochromatographic analysis method for making a positive or negative determination by causing a substance to be detected contained in a specimen to develop color by an immune reaction and confirming the developed color signal, wherein
   the substance to be detected is composed of a first substance to be detected, which is to be determined to be positive or negative, and a second substance to be detected, which is used as a standard for making the positive or negative determination by ascertaining the ratio of the second substance to be detected to the first substance to be detected, and the concentration of the first substance to be detected with respect to the second substance to be detected or the concentration of the first substance to be detected with respect to the total amount of the first substance to be detected and the second substance to be detected is used for making the positive or negative determination based on a specific concentration as the boundary, and
   in the determination method, the respective substances to be detected are individually caused to develop color,
   the concentration of the first substance to be detected at which the strengths of the developed color signals of the two substances are the same is set as the boundary for the determination, and
   the positive or negative determination of the first substance to be detected is made by confirming whether the strength of the developed color signal of the first substance to be detected is high or low with respect to the boundary.
2. The immunochromatographic analysis method as described in 1 above, wherein the strengths of the developed color signals of the first substance to be detected and the second substance to be detected are set to be the same by adjusting the using amount of a detection reagent which specifically reacts with the first substance to be detected.
3. An immunochromatographic analysis device for performing the method as described in 1 or 2 above for making a positive or negative determination by causing a substance to be detected contained in a specimen to develop color by an immune reaction and confirming the developed color signal, wherein
   the substance to be detected is composed of a first substance to be detected, which is to be determined to be positive or negative, and a second substance to be detected, which is used as a standard for making the positive or negative determination by ascertaining the ratio of the second substance to be detected to the first substance to be detected, and the concentration of the first substance to be detected with respect to the second substance to be detected or the concentration of the first substance to be detected with respect to the total amount of the first substance to be detected and the second substance to be detected is used for making the positive or negative determination based on a specific concentration as the boundary,
   the determination method includes a means for individually causing the respective substances to be detected to develop color,
   the concentration of the first substance to be detected at which the strengths of the developed color signals of the two substances are the same is set as the boundary for the determination, and
   the positive or negative determination of the first substance to be detected is made by confirming whether the strength of the developed color signal of the first substance to be detected is high or low with respect to the boundary.
4. An immunochromatographic analysis kit, comprising at least the immunochromatographic analysis device as described in 3 above and a developed color signal confirmation device capable of confirming the degrees of the strengths of the developed color signals of the first substance to be detected and the second substance to be detected at multiple concentrations.
5. The immunochromatographic analysis kit as described in 4 above, wherein the developed color signal confirmation device capable of confirming the degrees of the strengths of the developed color signals of the first substance to be detected and the second substance to be detected at multiple concentrations is a color sample.

### EFFECT OF THE INVENTION

According to the immunochromatographic analysis method and device of the present invention, a first substance to be detected, which is to be determined to be positive or negative, and a second substance to be detected, which is used as a standard for making a positive or negative determination by ascertaining the ratio of the second substance to be detected to the first substance to be detected, are individually caused to develop color by an immune reaction, and the concentration at which the strengths of the developed color signals of the two substances to be detected are the same is set as a boundary for the determination of the first substance to be detected, and therefore, the positive or negative determination of the first substance to be detected can be made without requiring expert knowledge of a specific device or analysis and also without involving complicated operations. Therefore, it has become possible to measure various components contained in various specimens such as blood and urine even in small-sized hospitals, at home, etc.

Further, according to the immunochromatographic analysis kit of the present invention, the strength of the developed color signal of the first substance to be detected can be compared with that of the developed color signal of the confirmation device, and therefore, the positive or negative determination of the first substance to be detected can be objectively made.

### BRIEF DESCRIPTION OF DRAWINGS

[FIG. 1] FIG. 1(a) and FIG. 1(b) are schematic views for explaining an immunochromatographic analysis kit utilizing the immunochromatographic analysis method and device of the present invention, and FIG. 1(a) is a cross-sectional view of the kit, and FIG. 1(b) is a plan view of the kit.
[FIG. 2] FIG. 2(a) to FIG. 2(d) are views for explaining a plurality of developed color signal confirmation devices prepared in the immunochromatographic analysis kit utilizing the immunochromatographic analysis method and device of the present invention.

### EMBODIMENTS FOR CARRYING OUT THE INVENTION

Hereinafter, the present invention will be described in more detail.

Examples of the specimen to be used in the present invention include blood samples such as blood, plasma, and serum, urine, saliva, spinal fluid, sweat, tear, amniotic fluid, nipple discharge fluid, nasal discharge, sputum, a nasal swab, a pharyngeal swab, exudate from the skin, and extracts from tissues, cells, and feces.

There are some cases where there are individual differences in the absolute amount of a substance to be detected which is to be determined to be positive or negative depending on the type of the substance, or cases where a positive or negative determination should be made based on the relative amount of the substance to be detected to another substance to be detected which is associated with the substance to be detected, and it is sometimes difficult to make a determination only by causing the substance to be detected to develop color and confirming the strength of the developed color. Therefore, with respect to such a substance to be detected, a positive or negative determination is made not by the absolute amount, but it is necessary to make a positive or negative determination based on the relative amount of the substance to be detected (first substance to be detected) to another substance to be detected (second substance to be detected) contained in one and the same specimen. For example, there are cases where even if the respective absolute amounts of the first substance to be detected and the second substance to be detected are within the range of the absolute amount of healthy individuals who are determined to be negative, the ratio of the first substance to be detected to the second substance to be detected is outside the range of healthy individuals, and therefore, the test subject should be determined to be positive (an unhealthy individual). In the present invention, the first substance to be detected and the second substance to be detected are caused to simultaneously develop color, and the ratio of the first substance to be detected to the second substance to be detected is ascertained.

Hereinafter, a description will be made by showing an example in which HbA1c is used as the first substance to be detected of the present invention, which is to be determined to be positive or negative, and HbA0 is used as the second substance to be detected, which is used as a standard for making the positive or negative determination by ascertaining the ratio of the second substance to be detected to the first substance to be detected, however, the present invention is not limited to the following example, and an analysis can be performed by using, for example, PSA (free PSA/total PSA ratio), cholesterol (LDL cholesterol/HDL cholesterol ratio) serving as the index of arteriosclerosis, a marker for hepatic dysfunction, nephrotic syndrome, or the like (albumin/globulin ratio), or the like as the substances to be detected other than the above-mentioned substances to be detected. That is, the present invention can be applied as long as the concentration of the first substance to be detected with respect to the second substance to be detected or the concentration of the first substance to be detected with respect to the total amount of the first substance to be detected and the second substance to be detected is used for making the positive or negative determination based on a specific concentration as the boundary.

The phrase "the second substance to be detected is used as a standard for making the positive or negative determination by ascertaining the ratio of the second substance to be detected to the first substance to be detected" means, for example, in the case where a positive determination is made when the amount of the first substance to be detected with respect to the total amount of the first substance to be detected and the second substance to be detected exceeds 6.0%, the signal strength is adjusted so that the strength of the developed color signal of the second substance to be detected which occupies 94.0% is the same as that of the developed color signal when the amount of the first substance to be detected is 6.0%.

In the present invention, first, HbA1c, which is to be determined to be positive or negative, and HbA0, which is to serve as a standard for making the positive or negative determination are individually caused to develop color by an immune reaction. A method for causing the substance to develop color is known and is disclosed in, for example, Patent Document 1. Specifically, the method can be performed by the following respective steps. First, blood is collected from a patient. Examples of a site where the blood is collected include a finger, a gum, an arm vein, and an ear.

An epitope of HbA1c in the blood collected is exposed on the surface of a hemoglobin protein. Examples of the method include a method in which hemoglobin is treated using a component for exposing the N-terminal of the hemoglobin β chain on the surface of the protein (N-terminal exposure agent), and a method in which hemoglobin is treated by being adsorbed onto a latex particle.

Examples of the exposure method using the N-terminal exposure agent include a method in which the blood is mixed with guanidine, thiocyanic acid, or a thiocyanate, a method in which the blood is mixed with any of a variety of surfactants, and a method in which the blood is mixed with these N-terminal exposure agents in combination.

Examples of the surfactant include sucrose monolaurate as a non-ionic surfactant.

Examples of an anionic surfactant include alkyl sulfates such as sodium lauryl sulfate, polyoxyethylene alkyl ether sulfates such as sodium polyoxyethylene lauryl ether sulfate, alkyl benzene sulfonates such as sodium dodecyl benzene sulfonate, acylamino acid salts such as lauroyl methyl alanine and sodium N-lauroyl sarcosine, sodium dialkyl sulfosuccinate, a sodium salt of β-naphthalene sulfonate formalin condensate, and a special polycarboxylic acid-based polymer surfactant.

Examples of a cationic surfactant include higher alkyl amines and quaternary ammonium salts. Examples of the quaternary ammonium salt include mono-long chain alkyl trimethyl ammonium salts, di-long chain alkyl dimethyl ammonium salts, benzalkonium chloride, benzethonium chloride, cetylpyridinium chloride, and dequalinium chloride.

Examples of an amphoteric surfactant include sulfobetaine 12-16, lauryl betaine, 2-alkyl-2-carboxymethyl-N-hydroxyethyl imidazolium betaine, lauryldimethylamine oxide, and lauric acid amidopropyl dimethylamino acetic acid betaine. Among these surfactants, two or more types may be used in combination.

It is preferred to use the N-terminal exposure agent as an aqueous solution, and the concentration of the N-terminal exposure agent is, for example, from 0.01 to 10 mass%. Further, the aqueous solution may contain a preservative such as sodium azide, a chelating agent such as EDTA, a metal ion such as NaCl or MgCl₂, or may contain a protein such as BSA or casein, or the like for the purpose of suppressing a non-specific reaction in the immunochromatographic analysis.

Further, the pH of the aqueous solution is preferably from 4.0 to 9.0, more preferably from 5.0 to 9.0, further more preferably from 6.0 to 8.0. In order to treat the blood using the aqueous solution, the aqueous solution may be added to a blood sample, followed by stirring. The temperature is preferably from 1°C to 50°C, more preferably from 10°C to 40°C, further more preferably from 15°C to 25°C. The treatment time is preferably from 10 seconds to 30 minutes, more preferably from 30 seconds to 5 minutes.

In this manner, a pretreated sample in which the glycated moiety of HbA1c in the blood sample is efficiently exposed on the surface of a hemoglobin protein is obtained.

Subsequently, an anti-hemoglobin antibody labeled with a labeling substance capable of specifically binding to hemoglobin in the pretreated sample prepared in the above step is reacted. Examples of such an antibody include a polyclonal antibody and a monoclonal antibody. The monoclonal antibody and the polyclonal antibody or fragments thereof are known and available, and can be prepared by a known method.

Examples of an animal species that produces the antibody include a human being, a mouse, a rat, a rabbit, and a goat. The immunoglobulin may be any of IgG, IgM, IgA, IgE, and IgD.

The monoclonal antibody is obtained according to a conventional method as follows. The spleen cells and myeloma cells of a mouse immunized with an antigen are fused, and a hybridoma which produces a desired antibody is selected, and a monoclonal antibody produced from this hybridoma is obtained (see, for example, the method of Kohler and Milstein [Nature, 256 (1975), 495-497]). The polyclonal antibody is obtained by separating a desired antibody from an antiserum obtained by immunizing an antibody-producing animal (for example, a human being, a mouse, a rat, a rabbit, a goat, a horse, etc.) with an antigen according to a conventional method.

As the labeling substance, a colored substance capable of visually confirming coloration is preferred, and a substance known in the art can be appropriately adopted. Examples thereof include colloidal metal particles, colloidal non-metal particles, colored latex, and an enzyme label, however, colloidal metal particles whose color hardly fades even if time has passed are particularly preferred from the viewpoint of stability of the label.

Examples of the colloidal metal particles include colloidal gold, platinum, copper, silver, and palladium, and other than these, particles obtained by mixing these colloidal metals. In particular, colloidal gold particles are preferred from the viewpoint that the particles having an appropriate particle diameter exhibit red color. The average particle diameter of the colloidal metal particles is, for example, from 1 to 500 nm, from the viewpoint of obtaining a strong color tone, from 10 nm to 150 nm, more preferably in the range of 20 to 100 nm.

Examples of the colloidal non-metal particles include colloidal selenium. The colloidal metal particles and the colloidal non-metal particles can be prepared according to a conventional method, and at this time, the particle diameter is adjusted so as to exhibit a desired color tone. In addition, it is also possible to use a commercially available product.

Examples of the colored latex include high-molecular weight polymer particles such as polystyrene particles colored with a coloring agent exhibiting red or blue color, and such colored latex can be prepared according to a conventional method. Examples of the enzyme label include peroxidase, alkaline phosphatase, glucose oxidase, and galactosidase. In the case where the enzyme label is used, a substrate for the enzyme and, according to need, a color-developing reagent are allowed to act on the enzyme, and the color developed by the reaction is detected.

Incidentally, the preparation of the antibody labeled with a labeling substance can be performed according to a known method. For example, as a method for supporting colloidal gold particles on an antibody, a known method such as physical adsorption or chemical binding can be employed. Specifically, for example, the antibody is added to a solution in which gold particles are colloidally dispersed and physically adsorbed on the particle, and thereafter, a blocking protein such as a bovine serum albumin solution is added thereto to block the surface of the particle to which the antibody is not bound, whereby the preparation can be performed.

In addition, for the antigen-antibody reaction, a known sandwich method, a competition method, a method combining these methods can be adopted.

Subsequently, in order to confirm the developed color signals derived from HbA1c and HbA0, HbA1c and HbA0 are, for example, immobilized on an anti-HbA1c antibody and an anti-HbA0 antibody, respectively. By doing this, it becomes possible to make a positive or negative determination based on the concentration of HbA1c in the blood. Incidentally, the anti-HbA1c antibody and the anti-HbA0 antibody may be either a monoclonal antibody or a polyclonal antibody as described above.

There are individual differences in blood components, and it is difficult to make the determination only by causing HbA1c to develop color and confirming the strength of the developed color. Therefore, it is necessary to cause HbA1c and HbA0 to simultaneously develop color and ascertain the ratio of HbA1c to HbA0. For example, in the case where a positive determination is made when the amount of HbA1c with respect to the total amount of HbA1c and HbA0 in the blood exceeds 6.0%, the strengths of the developed color signals of both HbA1c and HbA0 are adjusted so that the strength of the developed color signal when the amount of HbA1c is 6.0% is the same as that of the developed color signal of HbA0 which occupies 94.0%.

Examples of this adjustment method include a method in which the amount of a trapping substance to be labeled with a labeling substance for specifically reacting with and trapping each or both of the first and second substances to be detected is controlled, a method in which the amount of a trapping substance retained at a detection line in a membrane for specifically reacting with and trapping each or both of the first and second substances to be detected is controlled, a method in which the amount of a labeled trapping substance for specifically reacting with and trapping each or both of the first and second substances to be detected is controlled, and a method combining two or more methods among these.

Among these, a method in which the amount of a trapping substance retained at a detection line in a membrane for specifically reacting with and trapping each or both of the first and second substances to be detected is controlled, a method in which the amount of a labeled trapping substance for specifically reacting with and trapping each or both of the first and second substances to be detected is controlled, or a method combining these methods is preferred.

Here, the phrase "specifically reacting with both of the first and second substances to be detected" means that a substance shows high reactivity with both of the first and second substances to be detected and does not react with or shows low reactivity with substances other than the first and second substances to be detected, and therefore distinguishes the two substances to be detected from the other substances, and selectively reacts with the two substances to be detected.

In the case where a trapping substance which specifically reacts with both of the first and second substances to be detected is used, one and the same trapping substance may be used or two or more trapping substances having a different reactivity strength with the first and second substances to be detected may be used, however, the latter case in which two or more trapping substances are used is preferably used because it becomes easy to adjust the signal strength.

In the actual analysis, the developed color signals of HbA1c and HbA0 are compared, and in the case where the strength of the developed color signal of HbA1c is higher than that of HbA0, the amount of HbA1c is found to exceed 6.0%, and therefore a positive determination is made. On the other hand, in the case where the strength of the developed color signal of HbA1c is equal to or lower than that of HbA0, the amount of HbA1c is found to be 6.0% or less, and therefore a negative determination is made.

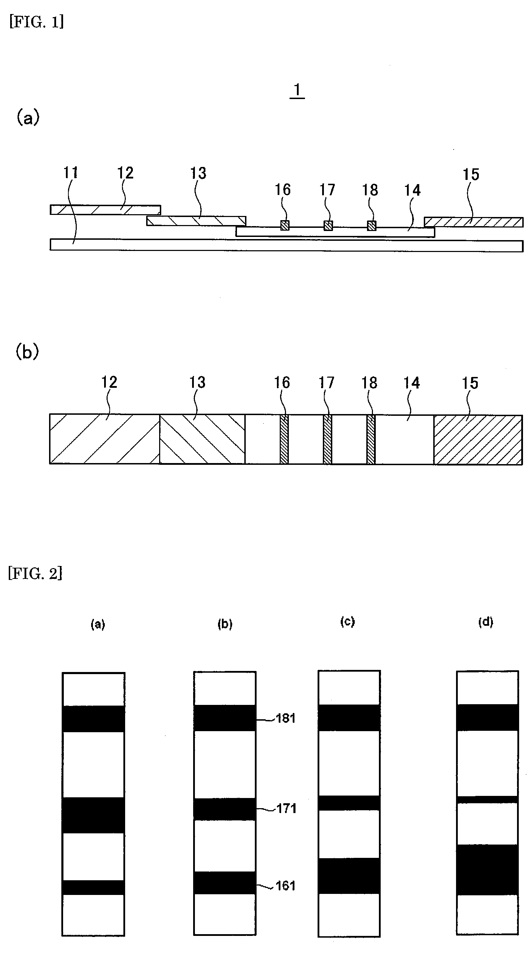

The immunochromatographic analysis method and device of the present invention are particularly preferably configured to be used as a kit. Hereinafter, the kit will be described. FIG. 1(a) and FIG. 1(b) are schematic views for explaining the immunochromatographic analysis kit utilizing the immunochromatographic analysis method and device of the present invention, and FIG. 1(a) is a cross-sectional view of the kit, and FIG. 1(b) is a plan view of the kit.

As shown in FIG. 1(a) and FIG. 1(b), an immunochromatographic analysis kit 1 includes a sample pad 12, a labeling substance-labeled anti-hemoglobin antibody-containing pad 13, an antibody-immobilized membrane 14, and a water-absorbing pad 15, which are provided on a plastic adhesive sheet 11 in this order along the longitudinal direction of the kit, respectively. Further, on the antibody-immobilized membrane 14, an anti-HbA1c antibody-coated portion 16 coated with an anti-HbA1c antibody, an anti-HbA0 antibody-coated portion 17 coated with an anti-HbA0 antibody, and an anti-IgG antibody-coated portion 18 coated with an anti-IgG antibody as a control are provided respectively.

The plastic adhesive sheet 11 serves as a base material of the kit, and one surface is made to serve as an adhesive surface by coating one surface with an adhesive or by sticking an adhesive tape to one surface, and part or the entire of the respective constituent parts described below are closely adhered onto the adhesive surface. As a material of the plastic adhesive sheet 11, a material which is impermeable to a sample and also is impermeable to moisture may be appropriately selected.

The sample pad 12 can be composed of a porous sheet having properties such that it quickly absorbs a sample, but has a low ability to retain the sample so that the sample promptly moves to a region where an antigen-antibody reaction occurs. Examples of the porous sheet include a cellulose filter paper, a glass fiber filter paper, polyurethane, polyacetate, cellulose acetate, nylon, and a cotton cloth.

On the anti-hemoglobin antibody-containing pad 13, the anti-hemoglobin antibody labeled with a labeling substance is retained, and examples of a material of the pad include glass fiber.

The antibody-immobilized membrane 14 may be any as long as it can absorb and move a sample specimen by a capillary phenomenon and can be composed of, for example, nitrocellulose, cellulose acetate, nylon, polyether sulfone, polyvinyl alcohol, polyester, glass fiber, polyolefin, cellulose, a mixed fiber thereof, or the like.

The anti-HbA1c antibody-coated portion 16, the anti-HbA0 antibody-coated portion 17, and the anti-IgG antibody-coated portion 18 provided on the antibody-immobilized membrane 14 are composed of, for example, a material capable of supporting and fixing each antibody, and examples of the material include nitrocellulose.

Examples of a material of the water-absorbing pad 15 include a material having an ability to promptly absorb an excess amount of a sample, and a glass filter paper or the like is used.

Next, the usage of the kit of the present invention will be described. First, as described above, a pretreated sample in which the glycated moiety of HbA1c in a blood sample is exposed on the surface of a hemoglobin protein is prepared, and this pretreated sample and a developing solution are dropped onto the sample pad 12.

The dropped pretreated sample and developing solution reach the anti-hemoglobin antibody-containing pad 13 by a capillary phenomenon and are subjected to an antigen-antibody reaction with the anti-hemoglobin antibody labeled with a labeling substance, whereby a complex is formed. The complex is then developed in the antibody-immobilized membrane 14 and reaches the anti-HbA1c antibody-coated portion 16, and HbA1c reacts with the anti-HbA1c antibody while it is passing through the portion and is immobilized thereon.

HbA0 and the developing solution do not react with the anti-HbA1c antibody-coated portion 16 and pass through the portion, however, when reaching the anti-HbA0 antibody-coated portion 17, HbA0 reacts with the anti-HbA0 antibody and is immobilized thereon. Incidentally, the other hemoglobin and water do not react therewith and move to the water-absorbing pad 15. In this manner, the developed color signals due to the presence of HbA1c and HbA0 can be confirmed in the respective coated portions.

Aside from this, in the kit of the present invention, a developed color signal confirmation device is prepared in advance. With the developed color signal confirmation device, the degrees of the strengths of the developed color signals of HbA1c and HbA0 at multiple concentrations can be confirmed.

For example, in the case where a positive determination is made when the amount of HbA1c with respect to the total amount of HbA1c and HbA0 in the blood exceeds 6.0% as described above, a photograph of the kit in which adjustment is made so that the strength of the developed color signal when the amount of HbA1c is 6.0% is the same as that of the developed color signal of HbA0 which occupies 94.0% or a color copy is prepared in advance as the developed color signal confirmation device to serve as a color sample.

In the actual analysis, as described above, the pretreated sample and the developing solution are developed by the kit, the developed color signals of HbA1c and HbA0 are compared, and in the case where the strength of the developed color signal of HbA1c is higher than that of HbA0, the amount of HbA1c is found to exceed 6.0%, and therefore a positive determination is made. On the other hand, in the case where the strength of the developed color signal of HbA1c is equal to or lower than that of HbA0, the amount of HbA1c is found to be 6.0% or less, and therefore a negative determination is made.

Incidentally, in this case, it is preferred to prepare in advance a plurality of developed color signal confirmation devices which show the developed color signal of HbA 1 c at each concentration.

FIG. 2(a) to FIG. 2(d) are views for explaining a plurality of developed color signal confirmation devices prepared. Incidentally, a black band in FIG. 2(a) to FIG. 2(d) indicates the degree of the strength of the developed color signal, and it means that as the black band is thicker, the strength of the developed color is higher, and as the black band is thinner, the strength of the developed color is lower.

FIG. 2(b) shows a color sample composed of a photograph of the kit in which adjustment is made so that the strength of a developed color signal 161 (a developed color signal from the anti-HbA1c antibody-coated portion 16 in FIG. 1(a) and FIG. 1(b)) when the amount of HbA1c is 6.0% is the same as that of a developed color signal 171 (a developed color signal from the anti-HbA0 antibody-coated portion 17 in FIG. 1(a) and FIG. 1(b)) of HbA0 which occupies 94.0% or a color copy.

Incidentally, 181 denotes a developed color signal of the control from the anti-IgG antibody-coated portion 18. Further, the kit also simultaneously includes a color sample when the amount of HbA1c is 5.5% as shown in FIG. 2(a), a color sample when the amount of HbA1c is 6.5% as shown in FIG. 2(c), and a color sample when the amount of HbA1c is 7.0% as shown in FIG. 2(d). According to this configuration, a positive or negative determination can be objectively made by visually comparing the degree of the developed color signal of a specimen with the degrees of the developed color signals of the respective color samples, and thus, this configuration is preferred.

Incidentally, as the above-mentioned developing solution, it is preferred to contain an anionic surfactant of a sulfonate having a hydrocarbon group with 10 to 30 carbon atoms at 0.01 to 0.25 w/v% from the viewpoint of suppressing the decrease in the strength of the developed color while exposing the glycated moiety of HbA1c.

### EXAMPLES

Hereinafter, the present invention will be further described by way of Examples and Comparative Examples, however, the present invention is not limited to the following examples.

### Preparation of kit

An immunochromatographic analysis kit 1 as shown in FIG. 1(a) and FIG. 1(b) was prepared according to the following procedure.

### (1) Preparation of anti-HbA1c antibody-coated portion 16, anti-HbA0 antibody-coated portion 17, and anti-IgG antibody-coated portion 18

As a membrane, a sheet composed of nitrocellulose (manufactured by Millipore, Inc., trade name: HF 12250 mm x 25 mm) was used. An anti-HbA1c monoclonal antibody, an anti-HbA0 monoclonal antibody, and an anti-IgG monoclonal antibody were diluted to 0.6 g/ml, 0.1 mg/ml, and 1.3 mg/ml, respectively, with a 10 mM phosphate buffer solution (pH 7.4) containing 5 mass% sucrose and 5 mass% isopropanol. The diluted solutions (150 µL) were applied to different places with a width of 1 mm on the membrane by an antibody applicator (manufactured by BioDot, Inc.), followed by drying at 50°C for 30 minutes and then drying at room temperature overnight, whereby an anti-HbA1c antibody-coated portion 16, an anti-HbA0 antibody-coated portion 17, and an anti-IgG antibody-coated portion 18 were provided, respectively, on an antibody-immobilized membrane 14.

### (2) Preparation of labeling substance solution

To 0.5 mL of a colloidal gold suspension (manufactured by Tanaka Kikinzoku Kogyo K.K., average particle diameter: 40 nm), 0.1 mL of a solution in which an anti-hemoglobin monoclonal antibody 1 which shows higher reactivity with HbA1c than with HbA0 or an anti-hemoglobin monoclonal antibody 2 which shows higher reactivity with HbA0 than with HbA1c was diluted to 0.1 mg/mL, respectively, with Tris buffer (pH 8.5) was added, and the resulting mixture was left to stand at room temperature for 10 minutes, whereby colloidal gold suspensions in which each of the two types of anti-hemoglobin monoclonal antibodies was supported on the colloidal gold were obtained. Then, to each of the suspensions, 0.1 mL of a Tris buffer solution (pH 8.5) containing 0.01 mass% PEG-SH (manufactured by NOF Corporation, trade name: SUNBRIGHT ME-200SH, molecular weight: 20,000) was added (PEG-SH concentration after addition: 0.001 mass%), and the resulting mixture was left to stand at room temperature for 10 minutes. Thereafter, the mixture was thoroughly stirred, and then centrifuged at 8000 x g for 15 minutes. After removing the supernatant, 0.1 mL of a phosphate buffer solution (pH 7.4) containing 1 mass% bovine serum albumin was added thereto, whereby a solution of the labeled anti-hemoglobin monoclonal antibody 1 and a solution of the labeled anti-hemoglobin monoclonal antibody 2 were obtained, respectively. A labeling substance solution was prepared by mixing 0.2 mL of the solution of the labeled anti-hemoglobin monoclonal antibody 1 and 0.02 mL of the solution of the labeled anti-hemoglobin monoclonal antibody 2. At this time, the amount of gold contained in the used solutions of the labeled anti-hemoglobin monoclonal antibody 1 and the labeled anti-hemoglobin monoclonal antibody 2 was 64 µg and 6.4 µg, respectively.

### (3) Preparation of Test Piece for Immunochromatography

A solution obtained by adding 100 µL of a phosphate buffer solution (pH 9.0) containing a 25 mass% aqueous solution of trehalose to 220 µL of the labeling substance solution prepared above was added uniformly to a 8 mm x 100 mm glass fiber pad (manufactured by Millipore, Inc.), followed by drying in a vacuum dryer, whereby an anti-hemoglobin antibody-containing pad 13 was prepared. Subsequently, on a plastic adhesive sheet 11, the thus prepared labeling substance-labeled anti-hemoglobin antibody-containing pad 13, and the antibody-immobilized membrane 14 were stuck, and a general-purpose sample pad 12 and a water-absorbing pad 15 were further stuck. Then, the resulting material was cut to a width of 5 mm by a cutter, whereby a test piece for immunochromatography was prepared.

A developing solution (aqueous solution) was prepared by stirring the respective components according to the formulation shown in the following Table 1.

**[Table 1]**

| Raw materials | Concentration |
|---|---|
| Triton X | 0.3% |
| Tween 20 | 1.0% |
| Bicine | 50 mM |
| Casein sodium | 2% |
| KCl | 80 mM |
| Microcide III | 0.8% |

In Table 1, "Triton X" is a name of a product manufactured by SIGMA, Inc., and the component thereof is octylphenoxypolyethoxyethanol.

"Tween 20" is a name of a product manufactured by Wako Pure Chemical Industries, Ltd., and the component thereof is polyoxyethylene sorbitan monolaurate.

"Bicine" is a name of a product manufactured by Dojindo Molecular Technologies, Inc., and the component thereof is N,N-bis(2-hydroxyethyl)glycine.

"Microcide III" is a name of a product manufactured by AMRESCO, Inc., and the component thereof is a mixture of 5-chloro-2-methyl-1,2-thiazol-3-one and 2-methyl-1,2-thiazol-3-one.

### Collection of specimen

Blood was collected by pricking the finger of each of healthy adult males and diabetic male patients, and a sample was prepared by mixing each blood sample in which the concentrations of HbA0 and HbA1c were measured by a latex agglutination method so that the concentration of HbA1c was 5.5%, 6.0%, or 7.0%.

### Preparation of pretreated sample

The blood and an N-terminal exposure agent (aqueous solution) having the formulation shown in the following Table 2 were stirred at room temperature for 0.5 minutes, and the resulting mixture was left to stand at room temperature for 2 minutes, whereby a pretreated sample was obtained. Incidentally, the N-terminal exposure agent was used in an amount 1,000 times the volume of the blood.

**[Table 2]**

| Raw materials | Concentration |
|---|---|
| SDS | 1.0% |
| EDTA | 5 mM |
| NaSCN | 100 mM |

### Implementation of immunochromatographic analysis

To the sample pad 12 of the test piece for immunochromatography prepared as described above, 10 µL of the pretreated sample and 100 µL of the developing solution were supplied, and after 10 minutes, the developed red color signal in the anti-HbA1c antibody-coated portion 16 and the anti-HbA0 antibody-coated portion 17 were visually confirmed.

### Preparation of developed color signal confirmation device

A pretreated sample was obtained by using HbA1c and HbA0 purified according to a conventional method and mixing with the N-terminal exposure agent having the formulation shown in the above Table 2. The pretreated sample and the developing solution were developed in the test piece for immunochromatography in the same manner as described above. The concentration of HbA1c with respect to the total amount of HbA1c and HbA0 was set to the following three concentrations: 5.5%, 6.0%, and 7.0%. Here, the concentration of HbA1c of 6.0% was set to the boundary for the positive or negative determination, and by a method in which the amount of a trapping substance retained at a detection line in a membrane for specifically reacting with and trapping each of the first and second substances to be detected is controlled, or a method in which the amount of a labeling substance used for labeling a trapping substance for specifically reacting with and trapping both of the first and second substances to be detected is controlled, the strengths of the developed color signals of HbA1c and HbA0 were adjusted to be the same. In detail, the preparation was performed as follows.

### [Method in which the amount of a trapping substance retained at a detection line in a membrane for specifically reacting with and trapping each of the first and second substances to be detected is controlled]

The preparation was performed in the same manner as in the preparation of the anti-HbA1c antibody-coated portion 16, the anti-HbA0 antibody-coated portion 17, and the anti-IgG antibody-coated portion 18 described above except that the anti-HbA0 antibody was diluted so that the dilution concentration of the anti-HbA0 antibody was changed as shown in Table 3, and the trapping substance for the second substance to be detected to be used in the antibody-immobilized membrane was controlled. The pretreated sample was developed along with the developing solution in the prepared test piece for immunochromatography, and after 10 minutes, the detection line of each of HbA1c and HbA0 was visually observed. The case where the strength of the developed color was lower than that of the other detection line was determined to be "low", the case where the strength of the developed color was higher than that of the other was determined to be "high", and the case where both have the same strength was determined to be "same". The determination results are shown in Table 3.

**[Table 3]**

| Concentration of HbA1c in pretreated sample | | Concentration of HbA1c: 5.5% | | | Concentration of HbA1c: 6.0% | | | Concentration of HbA1c: 7.0% | | |
|---|---|---|---|---|---|---|---|---|---|---|
| Concentration of anti-HbA0 monoclonal antibody (mg/mL) | | 0.05 | 0.1 | 0.3 | 0.05 | 0.1 | 0.3 | 0.05 | 0.1 | 0.3 |
| Determination | HbA1c line | same | low | low | high | same | low | high | high | same |
| | HbA0 line | same | high | high | low | same | high | low | low | same |

As shown in Table 3, in the case where the concentration of the diluted solution of the anti-HbA0 monoclonal antibody used in the antibody-immobilized membrane was controlled to be 0.1 mg/mL, the strengths of the developed colors of the detection lines of HbA1c and HbA0 were the same when the concentration of HbA1c was 6.0%.

### [Method in which the amount of a labeled trapping substance for specifically reacting with and trapping both of the first and second substances to be detected is controlled]

The preparation was performed in the same manner as in the preparation of the labeling substance solution described above except that the amount of the solution of the labeled anti-hemoglobin monoclonal antibody 2 was changed as shown in Table 4, and the amount of the labeled trapping substance for the second substance to be detected was controlled. The pretreated sample was developed along with the developing solution in the test piece for immunochromatography prepared using a labeling substance, and after 10 minutes, the detection line of each of HbA1c and HbA0 was visually observed, and determination was performed. The results are shown in Table 4.

**[Table 4]**

| Concentration of HbA1c in pretreated sample | | Concentration of HbA1c: 5.5% | | | Concentration of HbA1c: 6.0% | | | Concentration of HbA1c: 7.0% | | |
|---|---|---|---|---|---|---|---|---|---|---|
| Amount of solution of labeled anti-hemoglobin monoclonal antibody 2 (µL) | | 5 (1.6) | 20 (6.4) | 80 (25.2) | 5 (1.6) | 20 (6.4) | 80 (25.2) | 5 (1.6) | 20 (6.4) | 80 (25.2) |
| [amount of gold used for labeling (µg)] | | | | | | | | | | |
| Determination | HbA1c line | same | low | low | high | same | low | high | high | same |
| | HbA0 line | same | high | high | low | same | high | low | low | same |

As shown in Table 4, in the case where the amount of the solution of the labeled anti-hemoglobin monoclonal antibody 2 was controlled to be 20 µL, the strengths of the developed colors of the detection lines of HbA1c and HbA0 were the same when the concentration of HbA1c was 6.0%.

In addition, from the results shown in Table 3 and Table 4, it was confirmed that the strength of the developed color signal of the sample in which the concentration of HbA1c is lower than 6.0% is lower than that of the developed color signal of HbA0, and on the other hand, it was confirmed that the strength of the developed color signal of the sample in which the concentration of HbA1c is higher than 6.0% becomes higher than that of the developed color signal of HbA0 as the concentration of HbA1c becomes higher. The photographs of the respective obtained test pieces were taken and used as the color samples, and thus, the developed color signal confirmation devices were prepared.

As a result of observation and comparison between the test piece for immunochromatography in which the specimen of a healthy adult obtained above was developed and the color samples obtained above, it was found that the strength of the developed color signal of HbA1c of the healthy adult is lower than that of the developed color signal of HbA0, and the concentration of HbA1c in the blood of the healthy adult is less than 6.0%, and thus, a negative determination could be made.

On the other hand, as a result of observation and comparison between the test piece for immunochromatography in which the specimen of a diabetic patient was developed and the color samples obtained above, it was found that the strength of the developed color signal of HbA1c of the diabetic patient is higher than that of the developed color signal of HbA0, and corresponds to 7.0% in the color samples, and thus, a positive determination could be made.

While the present invention has been described in detail with reference to specific embodiments, it is apparent to those skilled in the art that various changes and modifications can be made without departing from the spirit and scope of the present invention. The present application is based on Japanese Patent Application (Japanese Patent Application No. 2014-004289) filed on January 14, 2014 and the entire contents of which are incorporated herein by reference.

### REFERENCE SIGNS LIST

- 1: immunochromatographic analysis kit
- 11: plastic adhesive sheet
- 12: sample pad
- 13: labeling substance-labeled anti-hemoglobin antibody-containing pad
- 14: antibody-immobilized membrane
- 15: water-absorbing pad
- 16: anti-HbA1c antibody-coated portion coated with anti-HbA1c antibody
- 17: anti-HbA0 antibody-coated portion coated with anti-HbA0 antibody
- 18: anti-IgG antibody-coated portion coated with anti-IgG antibody as control

## Claims

1. An immunochromatographic analysis method for making a positive or negative determination by causing a substance to be detected contained in a specimen to develop color by an immune reaction and confirming the developed color signal, wherein
the substance to be detected is composed of a first substance to be detected, which is to be determined to be positive or negative, and a second substance to be detected, which is used as a standard for making the positive or negative determination by ascertaining the ratio of the second substance to be detected to the first substance to be detected, and the concentration of the first substance to be detected with respect to the second substance to be detected or the concentration of the first substance to be detected with respect to the total amount of the first substance to be detected and the second substance to be detected is used for making the positive or negative determination based on a specific concentration as the boundary, and
in the determination method, the respective substances to be detected are individually caused to develop color,
the concentration of the first substance to be detected at which the strengths of the developed color signals of the two substances are the same is set as the boundary for the determination, and
the positive or negative determination of the first substance to be detected is made by confirming whether the strength of the developed color signal of the first substance to be detected is high or low with respect to the boundary.

2. The immunochromatographic analysis method according to claim 1, wherein the strengths of the developed color signals of the first substance to be detected and the second substance to be detected are set to be the same by adjusting the using amount of a detection reagent which specifically reacts with the first substance to be detected.

3. An immunochromatographic analysis device for performing the method according to claim 1 or 2 for making a positive or negative determination by causing a substance to be detected contained in a specimen to develop color by an immune reaction and confirming the developed color signal, wherein
the substance to be detected is composed of a first substance to be detected, which is to be determined to be positive or negative, and a second substance to be detected, which is used as a standard for making the positive or negative determination by ascertaining the ratio of the second substance to be detected to the first substance to be detected, and the concentration of the first substance to be detected with respect to the second substance to be detected or the concentration of the first substance to be detected with respect to the total amount of the first substance to be detected and the second substance to be detected is used for making the positive or negative determination based on a specific concentration as the boundary,
the determination method includes a means for individually causing the respective substances to be detected to develop color,
the concentration of the first substance to be detected at which the strengths of the developed color signals of the two substances are the same is set as the boundary for the determination, and
the positive or negative determination of the first substance to be detected is made by confirming whether the strength of the developed color signal of the first substance to be detected is high or low with respect to the boundary.

4. An immunochromatographic analysis kit, comprising at least the immunochromatographic analysis device according to claim 3 and a developed color signal confirmation device capable of confirming the degrees of the strengths of the developed color signals of the first substance to be detected and the second substance to be detected at multiple concentrations.

5. The immunochromatographic analysis kit according to claim 4, wherein the developed color signal confirmation device capable of confirming the degrees of the strengths of the developed color signals of the first substance to be detected and the second substance to be detected at multiple concentrations is a color sample.
